# EUROPEAN PATENT APPLICATION

(11) **EP 3 031 413 A1**
(43) Date of publication of application: **15.06.2016**
(21) Application number: 14382507.3
(22) Date of filing: 11.12.2014
(51) Int. Cl.: A61B 17/3213

(54) **Surgical scalpel**

(71) Applicant: Universitat Politècnica De Catalunya, 08034 Barcelona (ES); Mutua de Terrassa, Mutualitat de Previsio Social a Prima Fixa, 08221 Terrassa (Barcelona) (ES)
(72) Inventor: Alvarez del Castillo, Javier, 08034 Barcelona (ES); Alvarez del Castillo, Manuel, 08221 Terrassa - Barcelona (ES)
(74) Representative: Carvajal y Urquijo, Isabel

(57) **Abstract**

A surgical scalpel comprising: a blade (2) made up of a fastening stretch (21) and a cutting stretch (22); a handle (3) with a front end (31) and a rear end (32) and a joining mechanism (4) with a head-piece (5) joined to the front end (31), designed to fasten the blade (2) and facilitate the removal thereof. The fastening stretch (21) and cutting stretch (22) of the blade (2) are free from openings and the joining mechanism (4) is designed to put pressure on the fastening stretch (21) when holding the blade (2) and to release said pressure when the same is being extracted.

## Description

### Field of the invention

The present invention relates to a surgical scalpel, especially designed to prevent hairline cracks from forming in an interchangeable blade coupled to a corresponding handle.

### Background of the invention

Surgical techniques have changed considerably over the past few decades. However the basic instruments used to carry them out have not changed quite so much. Surgical incisions or cuts now constitute minimal invasions, leaving small openings though which to introduce surgical material. In contrast, these openings used to be much wider, in order to facilitate visibility and make it possible to manoeuvre the instruments used.

During surgical operations, surgeons currently use conventional scalpels or surgical knives to carry out those operations required of them in order to address the medical aim set out.

These surgeons manipulate the handles of scalpels in order to make the blade reach the place they need to cut, section or pierce. In complex operations, such as disc herniation or knee surgery, when it is difficult to access the area to be operated, surgeons are obliged to make movements with the scalpel that put pressure, such as bending moment, on the point of the knife, causing it to bend, despite the fact that scalpels are not mechanically designed for this.

Knives upon which such forces are exerted frequently break as a result of hairline cracks and thereby get stuck in areas of the patient's body that are difficult to access and delicate to locate, thus making it necessary to carry out subsequent operative procedures in order to extract the same and as a result, giving rise to intrinsic medical complications and the risks this poses to patients.

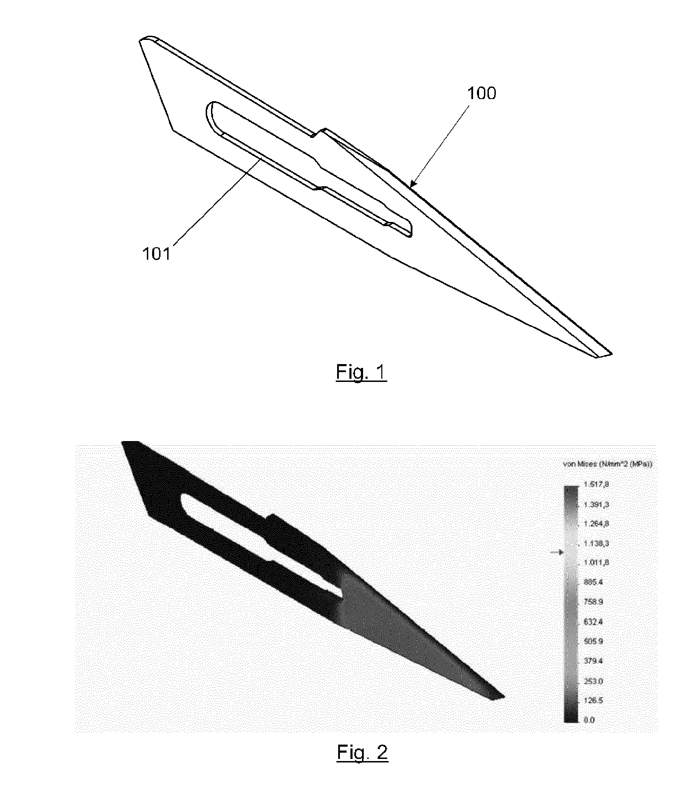

The conventional surgical knives or scalpels mentioned above mainly consist of an interchangeable blade, a handle and a joining mechanism, for joining the blade to the handle. In general, the blade is made up of a fastening stretch and a cutting stretch. The fastening stretch comprises one or more openings (for example grooves, holes, furrows or perforations, etc.,) needed to be able to fasten and extract the knife. The handle has a front end near to the blade and a rear end far away from the same. The joining mechanism usually has a head-piece joined to the front end of the handle, usually made up of an elongated extension of said end. In turn, said head-piece has at least one projection, prominence or protrusion, designed to fit closely into the opening or groove of the fastening stretch of the blade. Figures 1 and 2 show an interchangeable blade, pertaining to a conventional scalpel.

The fact that there is an opening in the profile of a conventional blade means that:
- During the manufacturing thereof: residual tension is produced at the edges of the opening of the blade as a result of being stamped with a die with a steel hoop and micro-fissures appear near to the opening or groove carved upon the die impacting the matrix hoop.
- Load: the appearance of concentrated tension around the edge of said opening, increasing the working tension to which the material of the blade is submitted two or threefold, in comparison to the same profile without carved grooves.
- During surgical use: overlapping residual tensions and micro-fissures caused by making the opening, alongside high amounts of tension concentrated at the edges of the opening as a result of use loads, produce hairline cracks in the end of the blade, when submitted to bending loads, commonly present in surgical operations of a certain degree of complexity.
- When changing the blade: it is not easy for health staff to manipulate the blade, since the opening therein and the prominence on the head-piece fit together very closely, such that considerable force must be applied to extract or couple said blade to the handle. This increases the risk of accidentally getting cut, which in turn increases the risk of catching contagious diseases and the number of accidents in hospitals and health centres.

The surgical scalpel of the present invention resolves the problem set out above, by means of a new configuration that makes it possible to use interchangeable blades without openings. Removing this opening eliminates residual tension and manufacturing micro-fissures from the work section of the blade and during usual use thereof, reduces the working tension to which the section of the blade is submitted, as a result increasing its mechanical resistance to hairline cracks in normal conditions of use. This configuration has a joining mechanism designed to put pressure on the blade when it is fastened, and to release this pressure when the same is extracted. As such, it is easier to insert and extract the blade, thereby removing the need to couple the opening to the protrusion in the head-piece and in turn, reducing the risk of accidents occurring. In addition, the handle was especially designed to facilitate the same being held comfortably, whilst reducing obstruction of the surgeon's visual field as much as possible. Finally, the surgical scalpel of the present invention is much simpler and cheaper to produce, since a die with a steel hoop is no longer used in the manufacturing process to form the inner opening of the blade.

### Description of the invention

The surgical scalpel of the present invention comprises:
- a blade formed by a fastening stretch and a cutting stretch,
- a handle with a front end and a rear end and
- a joining mechanism with a head-piece joined to the front end, configured to fasten the blade and make it possible to extract the same.

Said scalpel is characterised in that the fastening stretch and cutting stretch of the blade are free from holes and in that the joining mechanism is configured to put pressure on the fastening stretch when the blade is fastened and to release said pressure when the same is being extracted.

The head-piece is preferably substantially elliptical in shape, this improving the function and design of the scalpel. Said head-piece has a greater axis arranged in the direction of the longitudinal axis of the handle and a lesser axis, perpendicular to the same. The head-piece preferably comprises a flat front face, from which the cutting stretch of the blade extends, which facilitates the introduction of the blade.

The head-piece preferably comprises a first longitudinal groove designed to house the fastening stretch, which divides the head-piece into two opposite flexible portions. In order to have two blade fastening positions, the head-piece additionally comprises a second longitudinal groove, configured to house the fastening stretch, arranged crosswise from the first groove, where the first and second grooves divide the head-piece into four opposite flexible portions. Each flexible portion comprises a pressure surface, designed to put pressure on the fastening stretch. The head-piece comprises a neck joined to the front end of the handle, from which the flexible portions extend.

The joining mechanism preferably comprises a pressure element, designed to put pressure on the head-piece. Said pressure element is made up of a nut, designed to be screwed onto the front end of the handle and to grip the head-piece either partially or in whole.

The nut is designed to move axially along the length of the handle, comprising:
- a front stretch with a front passage, the internal diameter of which increases towards the head-piece,
- an intermediate stretch, with an intermediate passage with an internal thread and
- a rear stretch with a rear passage and a retention hole.

In turn, the head-piece comprises a maximum external diameter greater than the maximum internal diameter of the front passage of the nut, in such a way that the front stretch is designed to push the flexible portions against the fastening stretch of the blade when threading the nut and to release said push when unthreading the same.

In turn, the handle comprises an external thread on the front end that works in collaboration with the internal thread of the intermediate stretch, in order to make it possible to thread and unthread the nut. The handle comprises a widening around the perimeter of the rear end with an external diameter, forming a set between said external diameter relative to the internal diameter of the retention hole, which makes it possible to forcefully introduce the nut on the handle, preventing the non-forced extraction of the same from said handle, the nut therefore being retained in the handle.

An interchangeable blade for a surgical scalpel also forms an object to be protected under the present invention, the same being formed by a fastening stretch and a cutting stretch, where the fastening stretch and cutting stretch are free of openings. In other words, they do not have grooves, holes, furrows or perforations, designed to fasten the same to the handle of the scalpel.

### Brief description of the drawings

Below is a very brief description of a series of drawings, which serve to facilitate a better understanding of the invention and expressly relate to a preferred embodiment of said invention, presented as a non-limiting example of the same.
Figure 1 is a perspective view of a conventional blade for a surgical scalpel, with a carved groove, according to the current state of the art.
Figure 2 represents the distribution of tension in a conventional blade, as shown in Figure 1, upon being submitted to bending as a result of a side use load.
Figure 3 is a side view of the surgical scalpel of the present invention, according to a preferred embodiment.
Figure 4 is a plan view of the surgical scalpel shown in Figure 3.
Figure 5 is a piece-by-piece perspective view of the surgical scalpel shown in Figure 3.
Figure 6 is a side view of the blade of the present invention, according to the preferred embodiment.
Figure 7 is a perspective view of a cross-section of a detail of the front end of the handle of the present invention, according to a specific embodiment.
Figure 8 is a perspective view of a cross-section of a detail of the front end of the handle of the present invention, according to the preferred embodiment.
Figure 9 is a longitudinal cross-section of the surgical scalpel of the present invention, according to cut line A-A of Figure 4.
Figure 10 is a longitudinal cross-section of the nut of the present invention, according to the preferred embodiment.
Figure 11 represents a front detail of the head-piece of the present invention, according to the preferred embodiment.
Figure 12 represents a side detail - detail Z shown in Figure 9 - of the rear end of the handle of the present invention, according to the preferred embodiment.
Figure 13 represents the distribution of tension on the blade of the present invention, upon being submitted to bending as a result of a side use load.

### Detailed description of the invention

Figure 1 is a perspective view of a conventional surgical scalpel blade (100) according to the current state of the art. As can be seen, said blade (100) has an opening (101), which serves to fasten the same to the handle of the scalpel.
Figure 2 shows the distribution of tension in a conventional steel blade (100), like that shown in Figure 1, upon being submitted to bending as a result of a side use load of 6.16 N. The tension distribution was obtained by means of simulation, using the finite elements method.

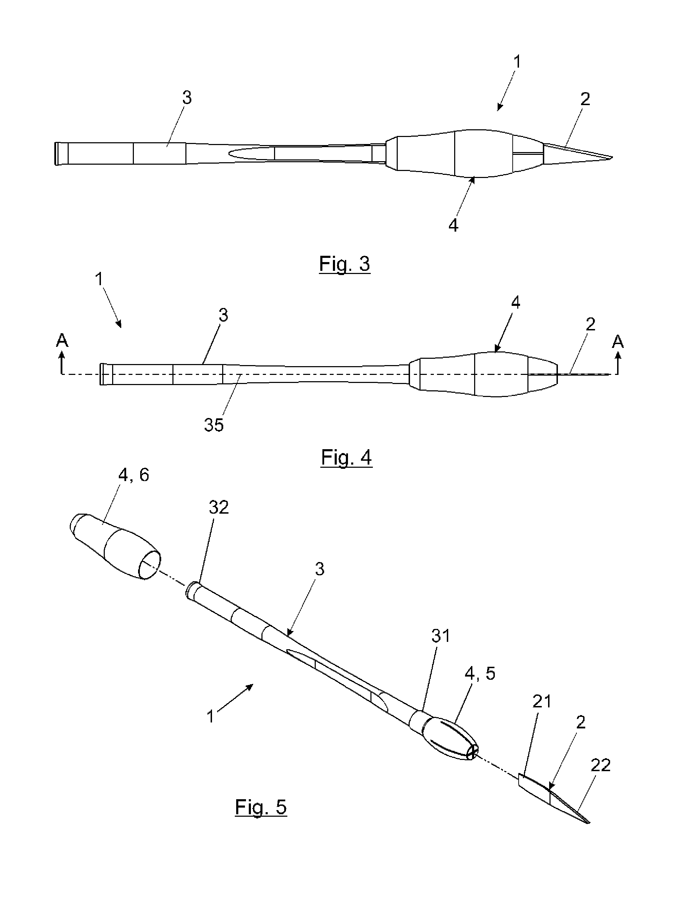

Figures 3, 4 and 5 are respective side, plan and piece-by-piece perspective views of the surgical scalpel (1) of the present invention, in accordance with the preferred embodiment. As can be seen, the surgical scalpel (1) comprises:
   - a blade (2) formed by a fastening stretch (21) and a cutting stretch (22),
   - a handle (3) with a front end (31) and a rear end (32) and
   - a joining mechanism (4) with a head-piece (5) joined to the front end (31), configured to hold the blade (2) and facilitate the extraction thereof.

The fastening stretch (21) and the cutting stretch (22) of the blade (2) are free of openings (101), i.e. do not have grooves, holes, furrows or perforations designed to fasten the same to the handle (3). In turn, the joining mechanism (4) is designed to put pressure on the fastening stretch (21) when fastening the blade (2) and to release this pressure when the same is being extracted.

The head-piece (5) is substantially elliptical in shape, thus improving the function and design of the scalpel (1), which has a greater axis arranged in the direction of the longitudinal axis (35) of the handle (3) and a lesser axis perpendicular to the same.

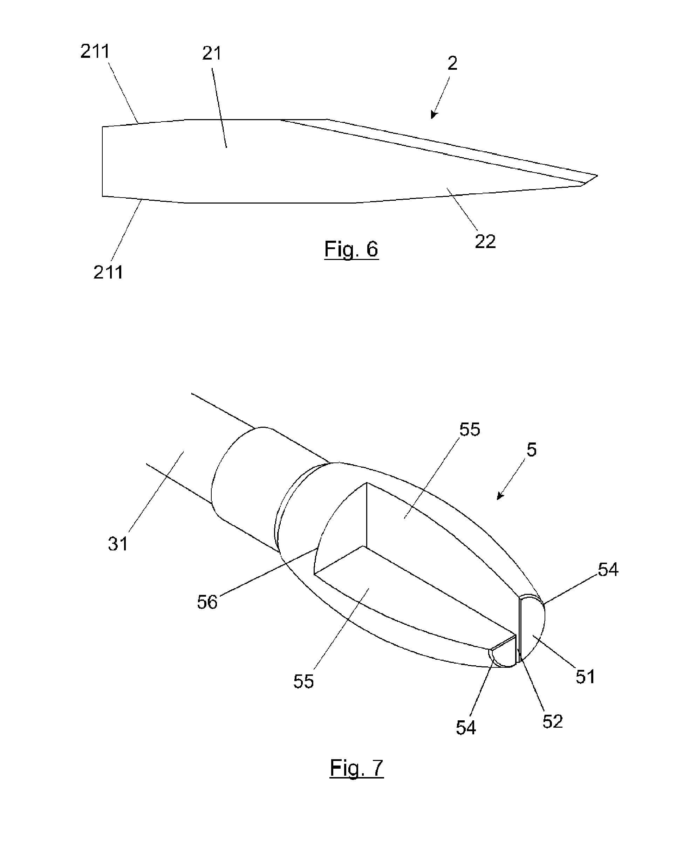

Figure 6 is a side view of the blade (2) of the present invention, according to the preferred embodiment. The disappearance of openings (101) removes residual tension and micro-fissures produced during manufacturing from the work section of the blade (2) and, during ordinary use thereof, reduces work tension to which it the blade (2) section is submitted to, consequently increasing its mechanical resistance.

The fastening stretch (21) comprises chamfers (211) to prevent the contour of the blade (2) from protruding sideways from the head-piece (5), given its substantially elliptical nature.

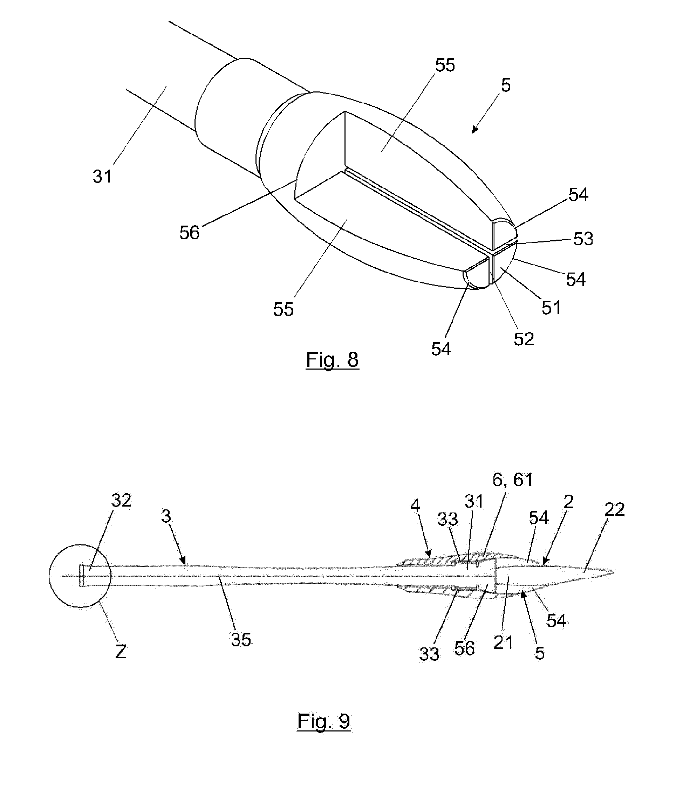

Figures 7 and 8 respectively show a cross-sectioned and perspective detail of the front end (31) of the handle (3) according to a particular embodiment and a cross-sectioned and perspective detail of the front end (31) of the handle (3) according to the preferred embodiment. For the purposes of illustrative clarity, the front upper quadrant of the head-piece (5) has not been represented. As can be seen, the head-piece (5) comprises a flat front face (51) from which the cutting stretch (22) of the blade (2) extends, which facilitates the introduction of the blade (2). Likewise, the head-piece (5) comprises a first longitudinal groove (52), designed to house the fastening stretch (21), which divides the head-piece (5) into two opposite flexible portions (54), see Figure 7. In order to provide two blade (2) fastening portions, the head-piece (5) additionally comprises a second longitudinal groove (53), designed to house the fastening stretch (21), arranged cross-wise from the first groove (52), where the first (52) and second (53) grooves divide the head-piece (5) into four opposite flexible portions (54), see Figure 8. Each flexible portion (54) comprises a pressure surface (55) configured to put pressure on the fastening stretch (21). The head-piece (5) comprises a neck (56) joined to the front end (31) of the handle (3), from which the flexible portions (54) extend.

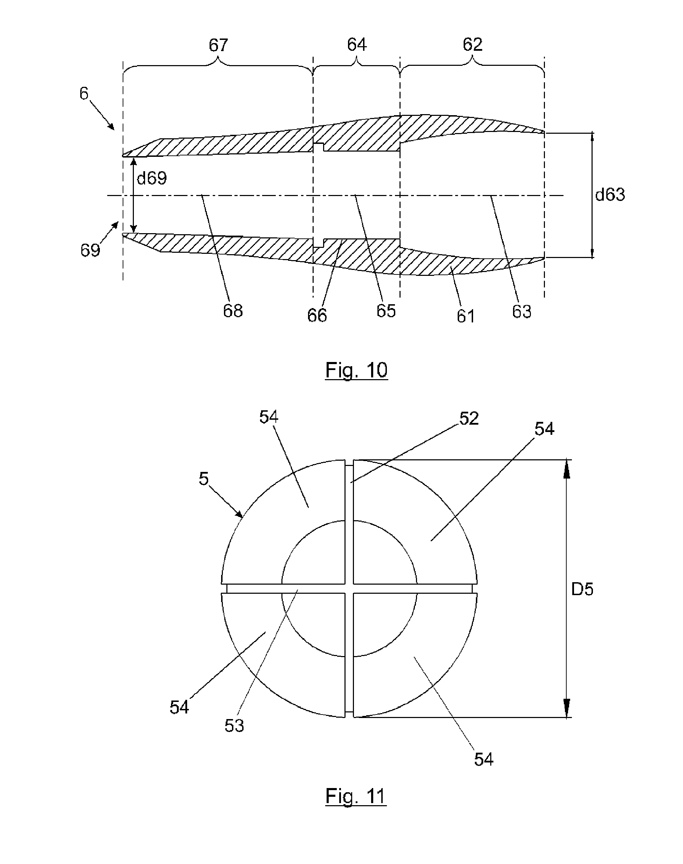

Figures 9 and 10 respectively show a longitudinal section of the surgical scalpel (1) according to cut line A-A of Figure 4 and a longitudinal section of the nut (61), according to the preferred embodiment. As can be seen, the joining mechanism (4) comprises a pressure element (6) designed to put pressure on the head-piece (5). Said pressure element (6) is formed by a nut (61) designed to thread into the front end (31) of the handle (3) and grip the head-piece (5) partially, see Figure 9.

The nut (61) is designed to move axially along the length of the handle (3), comprising:
- a front stretch (62), which has a front passage (63) with an internal diameter (d63) that increases towards the front part of the head-piece (5),
- an intermediate stretch (64), which has an intermediate passage (65) with an inner thread (66) and
- a rear stretch (67) with a rear passage (68) with a retention hole (69).

As can be seen in Figure 9, the handle (3) in turn comprises an external thread (33) at front end (32), which works in collaboration with the inner thread (66) of the intermediate stretch (64) in order to facilitate the threading and unthreading of the nut (61).

Figure 11 represents a front detail of the head-piece (5) of the present invention, according to the preferred embodiment. As can be seen, the head-piece (5) in turn comprises a maximum external diameter (D5) greater than the maximum internal diameter (d63) of the front passage (63), in such a way that the front stretch (62) is designed to push the flexible portions (54) against the fastening stretch (21) when threading the nut (61) and loosening said push when the same is being unthreaded.

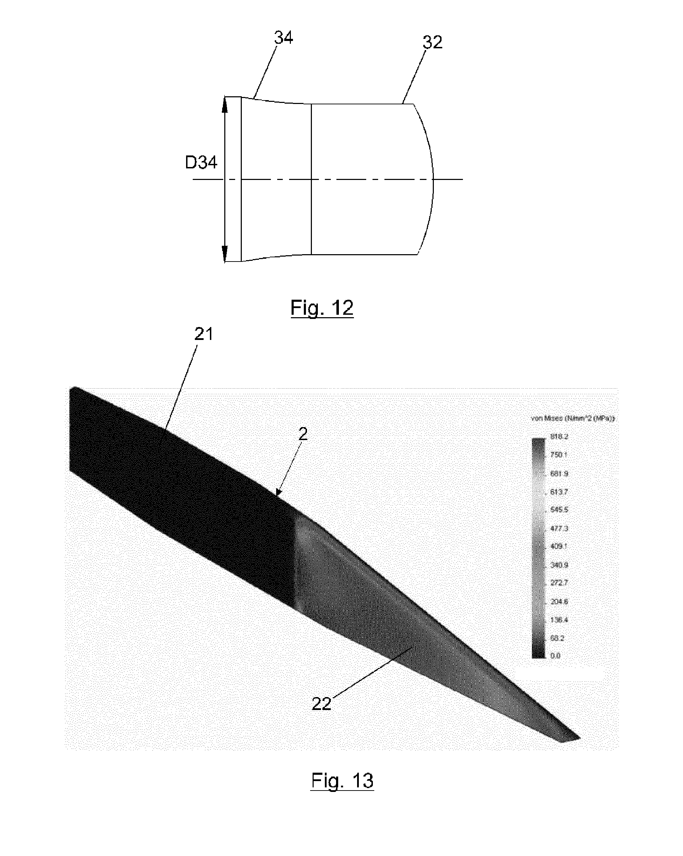

Figure 12 shows a side detail - detail Z of Figure 9 - at the rear end (31) of the handle (3) of the present invention, according to the preferred embodiment. As can be seen, the handle (3) comprises a widening around the perimeter (34) at the rear end (31) with an external diameter (D34), thereby establishing a set between said external diameter (D34) in relation to the internal diameter (d69) of the retention hole (69), which makes it possible to forcefully introduce the nut (61) into the handle (3) and prevents the non-forced extraction thereof from said handle (3).

Possible numerical values and materials to be used are listed below by way of example and correspond to a specific, non-limiting embodiment of the present invention. According to said example, it has a blade (2) manufactured using BOHLER UHB 20C carbon steel, 39mm in length and 6.2 mm high in the fastening stretch (21) and 0.4 mm thick, the fastening stretch (21) being 17.2 mm long. In turn, it has a handle (3), which measures 150 mm in length, made of stainless steel AISI 420, the front end (32) of which has an external thread (33) M8x1, in which the nut (61) is threaded in order to exert the pressure needed on the head-piece (5) that fastens the blade (2), thereby ensuring it is held adequately during surgical use. When the nut (61) is unthreaded, said pressure is released, in such a way that the blade (2) may easily be separated from the head-piece (5). At the front end (32), there is a head-piece (5) with an external diameter (D5) measuring 11.7 mm, whilst at the rear end (31), it has an external diameter (D34) of 7mm, with a dimensional tolerance f8 (-0.013, -0.035), which makes it possible to introduce the nut (61) but in turn prevents the involuntary extraction thereof. The nut (61) is made from stainless steel AISI 420, with a front passage (63) with a maximum internal diameter (d63) of 11.32 mm, as well as a rear passage (68) with a retention hole (69) with an internal diameter (d69) of 7mm, with a dimensional tolerance H8 (0.022, 0), which makes it possible to introduce the nut (61) in the handle (3) but once mounted, prevents the involuntary extraction thereof. The first groove (53) and second groove (54) are both 0.44 thick, just like the blade (2), and are 17mm deep. The grooves (52, 53) end against the neck (56) of the head-piece (5), perpendicularly to the longitudinal axis (35) of the handle (3), in order to ensure the blade (2) is positioned correctly.

Figure 13 shows the distribution of tension in the blade (2) of the present invention, upon being submitted to bending caused by a side use load of 6.16 N. The distribution of tension was obtained via simulation, using the finite elements method.

The trial blade (2) was manufactured from BOHLER UHB 20C carbon steel. Its geometry and dimensions are similar to those of the conventional blade (100) in Figures 1 and 2, measuring 39mm in length and 0.4 mm in thickness.

As can be seen in the comparison of tension distribution shown in Figures 2 and 13, the blade (2) of the present invention resists two times more bending tension than the traditional blade (100). This means that the blade (2) of the present invention can support bending forces two times higher than the bending forces conventional blades (100) are able to tolerate before breaking.

## Claims

1. A surgical scalpel, comprising:
• a blade (2) formed by a fastening stretch (21) and a cutting stretch (22),
• a handle (3) with a front end (31) and a rear end (32) and
• a joining mechanism (4) with a head-piece (5) joined to the front end (31) designed to hold the blade (2) and facilitate the extraction of the same,
said scalpel (1) being **characterised in that** the fastening stretch (21) and the cutting stretch (22) of the blade (2) are free from holes and **in that** the joining mechanism (4) is designed to put pressure on the holding section (21) when holding the blade (2) and to release said pressure when removing the same.

2. The surgical scalpel according to claim 1, **characterised in that** the head-piece (5) is substantially elliptical in shape.

3. The surgical scalpel according to any of the previous claims 1 to 2, **characterised in that** the head-piece (5) comprises a flat front face (51), from which the cutting stretch (22) of the blade (2) extends.

4. The surgical scalpel according to any of the previous claims 1 to 3, **characterised in that** the head-piece (5) comprises a first longitudinal groove (52) designed to house the fastening stretch (21), which divides the head-piece (5) into two opposite flexible portions (54).

5. The surgical scalpel according to claim 4, **characterised in that** the head-piece (5) comprises a second longitudinal groove (53) designed to house the fastening stretch (21), arranged cross-wise from the first groove (52), where the first (52) and second groove (53) divide the head-piece (5) into four opposite flexible portions (54).

6. The surgical scalpel according to any of the previous claims 4 to 5, **characterised in that** each flexible portion (54) comprises a pressure surface (55) designed to put pressure on the fastening stretch (21).

7. The surgical scalpel according to any of the previous claims 4 to 6, **characterised in that** the head-piece (5) comprises a neck (56) joined to the front end (31) of the handle (3), from which the flexible portions (54) extend.

8. The surgical scalpel according to any of the previous claims 1 to 7, **characterised in that** the joining mechanism (4) comprises a pressure element (6), designed to put pressure on the head-piece (5).

9. The surgical scalpel according to claim 8, **characterised in that** the pressure element (6) is made up of a nut (61) configured to thread onto the front end (31) of the handle (3) and to grip the head-piece (5) either partially or in whole.

10. The surgical scalpel according to claim 9, **characterised in that** the nut (61) is configured to travel axially along the length of the handle (3), comprising:
• a front stretch (62), which has a front passage (63) with an internal diameter (d63) that increases towards the head-piece (5);
• an intermediate stretch (64) with an intermediate passage (65) with an internal thread (66); and
• a rear stretch (67), which has a rear passage (68) with a retention hole (69).

11. The surgical scalpel according to any of the claims 7 and 10, **characterised in that** the head-piece (5) comprises a maximum external diameter (D5) greater than the maximum internal diameter (d63) of the front passage (63), in such a way that the front stretch (62) is designed to push the flexible portions (54) against the fastening stretch (21) when threading the nut (61) and to release said push when unthreading the same.

12. The surgical scalpel according to any of the claims 10 to 11, **characterised in that** the handle (3) comprises an external thread (33) at the front end (32), which works in collaboration with the internal thread (66) of the intermediate stretch (64), in order to facilitate the threading and unthreading of the nut (61).

13. The surgical scalpel according to any of the claims 10 to 12, **characterised in that** the handle (3) comprises a widening around the perimeter (34) at the rear end (31) with an external diameter (D34), a set being established between said external diameter (D34) in relation to the internal diameter (d69) of the retention hole (69), which facilitates the forced introduction of the nut (61) into the handle (3) and prevents the non-forced extraction of the same from said handle (3).

14. An interchangeable blade for a surgical scalpel, formed by a fastening stretch (21) and a cutting stretch (22), said blade (2) being **characterised in that** the fastening stretch (21) and cutting stretch (22) are free of openings.
